# EUROPÄISCHE PATENTSCHRIFT

(11) **EP 1 238 961 B1**
(45) Veröffentlichungstag und Bekanntmachung des Hinweises auf die Patenterteilung: **26.01.2005**
(21) Anmeldenummer: 02003273.6
(22) Anmeldetag: 22.02.2002
(51) Int. Cl.: C07C 67/24, C07C 69/78

(54) **Verfahren zur Herstellung von Hydroxybenzoesäurebenzylestern**
Process for the preparation of benzyl esters of hydroxybenzoic acids
Méthode de préparation d' esters benzyliques d'acides hydroxybenzoiques

(30) Priorität: 07.03.2001 DE 10110746
(43) Veröffentlichungstag der Anmeldung: 11.09.2002
(73) Patentinhaber: Bayer Chemicals AG, 51368 Leverkusen (DE)
(72) Erfinder: Ooms, Pieter, Dr., 47800 Krefeld (DE); Schenke, Bernd-Ulrich, 46236 Bottrop (DE)

(56) Entgegenhaltungen:
- WO-A-01/90044
- KUMARAPURAM N. PARAMESWARAN ET AL. : "O-Carbamoylsalicylates: Agents for modification of Hemoglobins" J. MED. CHEM., Bd. 30, 1987, Seite 936-939 XP001071247
- CHEN ET AL: "Structure-Activity Relationships in a Series of 5-[(2,5- Dihydroxybenzyl)amino]salicylate Inhibitors of EGF-Receptor- Associated Tyrosine Kinase: Importance of Additional Hydrophobic Aromatic Interactions" JOURNAL OF MEDICINAL CHEMISTRY, AMERICAN CHEMICAL SOCIETY. WASHINGTON, US, Bd. 37, Nr. 6, 1994, Seiten 845-859, XP002127746 ISSN: 0022-2623

## Beschreibung

Die vorliegende Erfindung betrifft ein Verfahren zur Herstellung von Hydroxybenzoesäurebenzylestem durch Umsetzung von Dibenzylether mit Alkylcarbonyloxy- oder Alkoxycarbonyloxybenzoesäure im Molverhältnis 1:1 bis 1:50 bei 10 bis 200°C und Drucken im Bereich von 0,1 bis 50 bar in Gegenwart von Säure als Katalysator.

Benzylsalicylat wird als Stabilisator in Duftkompositionen und Sonnenschutzmitteln eingesetzt.

Benzylsalicylat und Verfahren zu seiner Herstellung sind bereits bekannt.

So wird in der EP-A 11 75 02 beispielsweise die Herstellung von Benzylsalicylat durch Veresterung von Salicylsäure oder Umesterung von Salicylsäureestern mit Benzylalkohol beschrieben.

Benzylsalicylat kann auch durch Umsetzung von Alkalisalicylaten mit Benzylchlorid gegebenenfalls in Gegenwart von Phasentransferreagenzien hergestellt werden (JP 63/218652, EP-A 53 48 17). Nachteilig ist die Bildung von Salzen, die entsorgt werden müssen und somit die Wirtschaftlichkeit dieser Verfahren verringern.

Es bestand die Aufgabe, ausgehend von Dibenzylether ein Verfahren zur Herstellung von Hydroxybenzoesäurebenzylestem zu entwickeln, welches unter milden Reaktionsbedingungen durchführbar ist und kostengünstig zu guten Ausbeuten führt.

Es wurde ein Verfahren zur Herstellung von Hydroxybenzoesäurebenzylestern der Formel 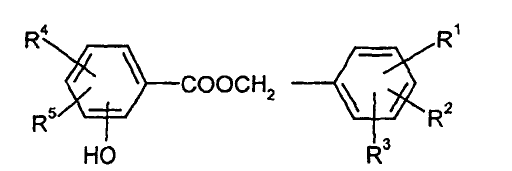 worin
R¹ bis R⁵ gleich oder verschieden sind und für C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen stehen,
aus Dibenzylethem gefunden, das dadurch gekennzeichnet ist, dass Dibenzylether der Formel 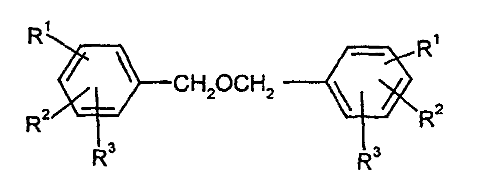 worin
R¹, R² und R³ die oben genannte Bedeutung haben,
oder Gemische aus Dibenzylethern und Benzylalkoholen der Formel 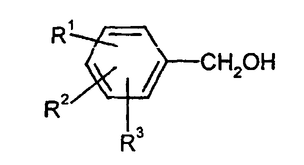 worin
R¹, R² und R³ die oben genannte Bedeutung haben,
mit Alkylcarbonyloxy- oder Alkoxycarbonyloxybenzoesäuren der Formel 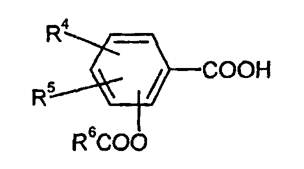 worin
R⁴ und R⁵ die oben genannte Bedeutung haben und
R⁶ für Wasserstoff oder eine geradkettige, verzweigte, gesättigte oder ungesättigte, gegebenenfalls mit Halogen substituierte Alkyl-, Aralkyl-, Aryl-, Alkoxy-, Aralkoxyund Aryloxygruppe mit 1 bis 50 C-Atomen steht,
in Gegenwart einer oder mehrerer Säuren als Katalysator, umgesetzt werden.

Das erfindungsgemäße Verfahren läßt sich kostengünstig und unter milden Reaktionsbedingungen durchführen.

Die Reste R¹ bis R⁵ haben im Allgemeinen die folgende Bedeutung:

Alkyl bedeutet im allgemeinen einen geradkettigen oder verzweigten Kohlenwasserstoffrest mit 1 bis 6, bevorzugt 1 bis 4, im besonderen bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl und Ethyl.

Alkoxy bedeutet im allgemeinen einen geradkettigen oder verzweigten Alkoxyrest mit 1 bis 6, bevorzugt 1 bis 4, im besonderen bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy und Ethoxy.

Halogenalkyl bedeutet im Allgemeinen einen geradkettigen oder verzweigten Kohlenwasserstoffrest mit 1 bis 6, bevorzugt 1 bis 4, im besonderen bevorzugt 1 oder 2 Kohlenstoffatomen mit 1 bis 10, bevorzugt 1 bis 8, im besonderen bevorzugt 1 bis 5 Halogenatomen. Beispielsweise seien Chlormethyl, Fluormethyl, Trifluormethyl, Fluorethyl, Fluorpropyl und Hexafluorbutyl genannt. Bevorzugt sind Fluormethyl und Trifluormethyl.

Halogenalkoxy bedeutet im Allgemeinen einen geradkettigen oder verzweigten Alkoxyrest mit 1 bis 6, bevorzugt 1 bis 4, im besonderen bevorzugt 1 oder 2 Kohlenstoffatomen mit 1 bis 10, bevorzugt 1 bis 8, im besonderen bevorzugt mit 1 bis 5 Halogenatomen. Beispielsweise seien Chlormethoxy, Fluormethoxy, Trifluormethoxy, Fluorethoxy, Fluorpropoxy und Hexafluorbutoxy genannt. Bevorzugt sind Fluormethoxy und Trifluormethoxy.

Halogen bedeutet im Allgemeinen Fluor, Chlor, Brom und Jod, bevorzugt Fluor und Chlor.

Bevorzugte Substituenten für R¹ bis R⁵ sind Methyl, Trifluormethyl, Methoxy, Fluor oder Chlor.

Alkyl-, Aralkyl-, Aryl-, Alkoxy-, Aralkoxy- und Aryloxygruppen (R⁶) enthalten im allgemeinen 1 bis 50 C-Atomen, bevorzugt 1 bis 10 Kohlenstoffatome.

Nach dem erfindungsgemäßen Verfahren können beispielsweise die folgenden Hydroxybenzoesäurebenzylestern hergestellt werden: 2-Hydroxybenzoesäurebenzylester (Salicylsäurebenzylester); 3-Hydroxybenzoesäurebenzylester, 4-Hydroxybenzoesäurebenzylester; 3-Chlor-2-hydroxybenzoesäurebenzylester, 4-Chlor-2-hydroxybenzoesäurebenzylester, 5-Chlor-2-hydroxybenzoesäurebenzylester; 6-Chlor-2-hydroxybenzoesäurebenzylester, 3-Brom-2-hydroxybenzoesäurebenzylester, 3-Fluor-2-hydroxybenzoesäurebenzylester, 4-Fluor-2-hydroxybenzoesäurebenzylester, 2-Fluor-3-hydroxybenzoesäurebenzylester, 2-Fluor-4-hydroxybenzoesäurebenzylester; 3-Fluor-2-hydroxybenzoesäurebenzylester, 2-Fluor-5-hydroxybenzoesäurebenzylester, 2-Fluor-6-hydroxybenzoesäurebenzylester, 2-Hydroxy-3-methylbenzoesäurebenzylester, 2-Hydroxy-4-methylbenzoesäurebenzylester, 3-Hydroxy-2-methylbenzoesäurebenzylester, 4-Hydroxy-2-methylbenzoesäurebenzylester, 2-Fluor-6-hydroxy-4-methoxybenzoesäurebenzylester, 3-Trifluormethyl-2-hydroxybenzoesäurebenzylester, 4-Trifluormethyl-2-hydroxybenzoesäurebenzylester, 2-Trifluormethyl-3-hydroxybenzoesäurebenzylester, 2-Fluorethyl-4-hydroxybenzoesäurebenzylester und 4-Fluorbutyl-2-hydroxybenzaesäurebenzylester.

Bei dem im erfindungsgemäßen Verfahren eingesetzten Dibenzylether handelt es sich um unsubstituierte oder einen substituierten Dibenzylether.

Besonders bevorzugt wird unsubstituierter Dibenzylether eingesetzt.

In das erfindungsgemäßen Verfahren können Dibenzylether oder Dibenzylether/-Benzylalkoholgemische, wie sie beispielsweise bei der Herstellung von Benzylalkohol aus Benzylchlorid anfallen, eingesetzt werden. Der Gehalt an Dibenzylether in dem Gemisch kann 50 bis 100 Gew.%, bevorzugt 60 bis 99 Gew.%, besonders bevorzugt 70 bis 98 Gew.% sein.

Für das erfindungsgemäße Verfahren seien beispielsweise die folgenden Alkylcarbonyloxy- und Alkoxycarbonyloxybenzoesäuren genannt:
2-Formyloxybenzoesäure, 3-Formyloxybenzoesäure, 4-Formyloxybenzoesäure, 2-Acetoxybenzoesäure, (2-Acetylsalicylsäure); 3-Acetoxybenzoesäure, 4-Acetoxybenzoesäure, 2-Propionyloxybenzoesäure, 2-Butyryloxybenzoesäure, 2-Benzoyloxybenzoesäure, 2-Acetoxy-3-chlorbenzoesäure, 2-Acetoxy-4-chlorbenzoesäure, 2-Acetoxy-5-chlorbenzoesäure, 2-Acetoxy-6-chlorbenzoesäure, 2-Acetoxy-3-brombenzoesäure, 2-Acetoxy-3-chlorbenzoesäure, 2-Formyloxy-3-fluorbenzoesäure, 2-Acetoxy-3-fluorbenzoesäure, 2-Acetoxy-4-fluorbenzoesäure, 3-Acetoxy-2-fluorbenzoesäure, 2-Fluor-4-propionyloxybenzoesäure, 2-Butyroxy-3-fluorbenzoesäure, 2-Fluor-5-hydroxybenzoesäure, 6-Acetoxy-2-fluorbenzoesäure, 2-Acetoxy-3-methylbenzoesäure, 2-Acetoxy-4-methylbenzoesäure, 3-Acetoxy-2-methylbenzoesäure, 4-Acetoxy-2-methylbenzoesäure, 6-Acetoxy-2-fluor-4-methoxybenzoesäure, 2-Acetoxy-3-trifluormethylbenzoesäure, 2-Acetoxy-4-trifluormethylbenzoesäure, 3-Acetoxy-2-trifluormethylbenzoesäure, 4-Acetoxy-2-fluorethylbenzoesäure, 2-Acetoxy-4-fluorbutyl-benzoesäure, 2-Methoxycarbonyloxybenzoesäure, 2-Ethoxycarbonyloxybenzoesäure, 3-Methoxycarbonyloxybenzoesäure oder Trifluoracetoxybenzoesäure.
Bevorzugt sind Alkylcarbonyloxy- und Alkoxycarbonyloxybenzoesäuren mit 2 bis 30 C-Atomen, besonders bevorzugt 2 bis 12 C-Atomen. Ganz besonders bevorzugt sind Alkylcarbonylsalicylsäuren.

Das erfindungsgemäße Verfahren wird vorzugsweise unter Entfernung des gebildeten Wassers durchgeführt. Geeignet ist die Entfernung des Wassers durch Destillation oder durch Durchleiten eines inerten Gases wie beispielsweise Stickstoff. Bevorzugt werden zur Entfernung des gebildeten Wassers wasserentziehende Mittel eingesetzt, beispielsweise Zeolithe, Aluminiumoxide oder Tonerden. Besonders bevorzugt wird das gebildete Wasser dadurch entfernt, dass man die Umsetzung in Gegenwart des entsprechenden Anhydrids der eingesetzten Alkylcarbonyloxy- oder Alkoxycarbonyloxybenzoesäure als wasserentziehendes Mittel durchführt. Ganz besonders bevorzugte Anhydride sind Acetylsalicylsäureanhydride.

Im erfindungsgemäßen Verfahren werden vorzugsweise 0,5 bis 50 mol an Alkylcarbonyloxy- oder Alkoxycarbonyloxybenzoesäure, bevorzugt 1 bis 30 mol, besonders bevorzugt 2 bis 20 mol, bezogen auf Dibenzylether, eingesetzt.

Wird das erfindungsgemäße Verfahren in Gegenwart des entsprechenden Anhydrids der eingesetzten Alkylcarbonyloxy- oder Alkoxycarbonyloxybenzoesäure durchgeführt, so werden vorzugsweise 0,1 bis 10 mol Anhydrid, bevorzugt 0,5 bis 7,5 mol, besonders bevorzugt 1 bis 5 mol, bezogen auf Dibenzylether, eingesetzt. Da ein Molekül eingesetztes Anhydrid unter Wasseraufnahme zu 2 Molekülen Carbonsäure abreagiert, können im erfindungsgemäßen Verfahren geringere Mengen an Carbonsäure eingesetzt werden. Es werden dann vorzugsweise 0,25 bis 25 mol Carbonsäure, bevorzugt 0,5 bis 15 mol, besonders bevorzugt 1 bis 10 mol Carbonsäure, bezogen auf Dibenzylether, eingesetzt.

Im erfindungsgemäßen Verfahren werden Säuren sowohl als homogene als auch als heterogene Katalysatoren eingesetzt.

Die als Katalysator für das erfindungsgemäße Verfahren eingesetzten Säuren haben im Allgemeinen einen p_{H} Wert von 1 bis 6, bevorzugt von 1 bis 5, insbesondere bevorzugt von 1 bis 4.

Geeignete Katalysatoren für das erfindungsgemäße Verfahren sind anorganische Säuren wie beispielsweise Schwefelsäure, Chlorwasserstoff, Bromwasserstoff, Jodwasserstoff, Flusssäure, Perchlorsäure, Chlorsulfonsäure oder Phosphorsäure, organische Säuren wie beispielsweise Trifluoressigsäure, Methansulfonsäure, Ethansulfonsäure, Benzolsulfonsäure, 4-Toluolsulfonsäure oder Trifluormethansulfonsäure und Lewissäuren wie beispielsweise Bortrifluorid, Aluminiumchlorid, Aluminiumbromid, Aluminiumiodid, Zinkchlorid, Zinnchlorid, Titanchlorid oder Zirkonchlorid.

Bevorzugt sind Schwefelsäure, Trifluormethansulfonsäure, 4-Toluolsulfonsäure und Bortrifluorid, besonders bevorzugt Schwefelsäure, Trifluormethansulfonsäure und Bortrifluorid.

Die Säuren können sowohl als homogener als auch auf einem inerten Träger als heterogener Katalysator eingesetzt werden.

Im erfindungsgemäßen Verfahren werden als heterogene saure Katalysatoren vorzugsweise saure Ionenaustauscher wie beispielsweise sulfonylierte Polymere wie sulfonylierte Polystyrole, sulfonylierte Styrol-Divinylbenzol-Copolymerisaten und sulfonylierte Phenolformaldehyd-Harzen eingesetzt.

Bevorzugt sind sulfonylierte Polystyrole und sulfonylierte Styrol-Divinylbenzol-Copolymerisate, besonders bevorzugt sulfonylierte Polystyrole.

Die Ionenaustauscher sind durch Umsetzung von Polystyrol oder Styrol-Divinylbenzol-Copolymerisaten mit Sulfonierungsmitteln wie Schwefelsäure oder Chlorsulfonsäure zugänglich.

Methoden zur Herstellung sind gut bekannt und beispielsweise beschrieben in Encyclopedia of Polymer Science and Technology, 1967, Band 7, S. 692 bis 705.

Geeignet sind handelsübliche Ionenaustauscher wie beispielsweise Produkte vertrieben unter den registrierten Handelsnamen Lewatit, Amberlyst, Amberlite, Dowex, Duolite, Nafion, Permutit, Chempro und Imac.

Die Ionenaustauscher können in Kugelform vorliegen und Korngrößen von 0,3 bis 3,0 mm Durchmesser aufweisen. Sie können vom Geltyp oder makroporös sein. Ihre Totalkapazität an Säurefunktionen in wasserfeuchter Form mit einem Wassergehalt von ca. 75 bis 85 Gew.-% reicht von 0,7 bis 2,1 mval/ml Ionenaustauscher oder von 3,5 bis 5 mval/ml, bezogen auf 1 g Trockensubstanz an Ionenaustauscher.

Diese Ionenaustauscher werden gegebenenfalls durch Wärme, gegebenenfalls im Vakuum, gegebebenfalls durch Waschen mit hydrophilen organischen Flüssigkeiten wie beispielsweise die eingesetzte Carbonsäure oder das eingesetzte Carbonsäureanhydrid oder gegebenenfalls durch azeotrope Destillation mit organischen Flüssigkeiten wie Toluol, Xylol oder Methylenchlorid getrocknet

Die genannten Ionenaustauscher werden bei Arbeiten mit suspendierten Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierliche Fahrweise im Gegen- oder Gleichstrom oder in der Rieselphase am Festbettkatalysator werden Katalysatorbelastungen von 0,05 g bis 5000 g Dibenzylether pro Liter aufgequollenem Ionenaustauscher pro Stunde, bevorzugt von 0,1 bis 4000 g/l h und besonders bevorzugt von 1,0 bis 3000 g/l h verwendet.

In einer besonderen Ausführungsform des erfindungsgemäßen Verfahren werden als Katalysatoren Heteropolysäuren (Polyoxymetallate) der allgemeinen Formel

Hₐ X_{b} M_{c} O_{d}

worin
- H: für Wasserstoff und/oder Metallkationen steht,
- X: für Phosphor, Silizium, Bor oder Germanium steht,
- M: für Wolfram, Molybden, Vanadium oder Chrom steht und
- a: für die Zahlen 3, 4, 5 oder 6 steht, mit der Maßgabe, dass die Elektroneutralität der Heteropolysäuren oder deren Salze gegeben ist,
- b: für die Zahlen 1 oder 2 steht,
- c: für die Zahlen 12 oder 18 steht und
- d: für die Zahlen 40 oder 62 steht,
eingesetzt.

Als Metallkatione seien Alkalimetalle wie Lithium, Natrium, Kalium, Rubidium, Caesium oder Mangan, Nickel, Kobalt, Kupfer oder Lanthan genannt.

Bevorzugt sind Heteropolysäuren des Keggin-Typs ( Chem. Rev. 98 (1998) S. 12 d.h. Verbindungen mit b = 1, c = 12 und d = 40, wobei X für Wasserstoff steht.

Bevorzugt sind Molybden-, Wolfram- und Vanadiumoxiden mit Phosphorsäure oder Kieselsäure, wie beispielsweise Wolframatophosphorsäure, Wolframatokieselsäure, Molybdatophosphorsäure, Molybdatokieselsäure, Vanadatophosphorsäure und Vanadatokieselsäure, besonders bevorzugt sind Wolframatophosphorsäure, Wolframatokieselsäure, Molybdatophosphorsäure und Molybdatokieselsäure.

Methoden zur Herstellung sind gut bekannt und beispielsweise beschrieben in "Römpp Lexikon Chemie, 10. Auflage, Stuttgart/New York, 1997, Band 3, S. 1741 und Chemical Reviews 98, (1998), S. 3-49, Catal. Rev.-Sci. Eng., 37, 311 bis 321 (1995).

Die Heteropolysäuren gegebenenfalls deren Hydrate, können sowohl als homogener als auch auf einem inerten Träger als heterogener Katalysator eingesetzt werden.

Die Katalysatoren können z.B. als Pulver oder Formkörper eingesetzt werden und nach der Umsetzung z.B. durch Filtration, Sedimentation oder Zentrifugieren abgetrennt werden.

Für den Fall der Anordnung als Festbett werden die Katalysatoren vorzugsweise auf einem Träger aufgebracht und als Formkörper, z.B. als Kugeln, Zylinder, Stäbchen, Hohlzylinder, Ringe usw. eingesetzt.

Als Trägermaterial geeignet sind Aktivkohle, Kieselgel, Aluminiumoxid, Alumosilikate wie Zeolithe oder Schichtsilikate, Tonerden, Titanoxide und Zirkonoxide.

Diese heterogenisierte Katalysatoren werden gegebenenfalls durch Wärme, gegebenenfalls im Vakuum, gegebebenfalls durch Waschen mit hydrophilen organischen Flüssigkeiten wie beispielsweise die eingesetzte Carbonsäure oder das eingesetzte Carbonsäureanhydrid oder gegebenenfalls durch azeotrope Destillation mit organischen Flüssigkeiten wie Toluol, Xylol oder Methylenchlorid getrocknet.

Die Säuren werden bei Arbeiten mit suspendierten Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierliche Arbeitsweise im Gegen- oder Gleichstrom oder in der Rieselphase am Festbettkatalysator werden Katalysatorbelastungen von 0,05 g bis 5000 g Dibenzylether pro Liter immobilisierten Säure pro Stunde, bevorzugt von 0,1 bis 4000 g/l h und besonders bevorzugt von 1,0 bis 3000 g/l h verwendet.

Vorzugsweise wird das erfindungsgemäße Verfahren unter intensiver Durchmischung der Reaktionspartner durchgeführt. Eine intensive Durchmischung kann auf verschiedene, dem Fachmann bekannte Weisen, etwa durch Rühren, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 568-570, Verlag Chemie, Weinheim 1988 näher beschrieben.

Eine bevorzugte Ausführungsform des erfindungsgemäßen Verfahrens ist indem man in eine Mischung oder Suspension von Säure, gegebenenfalls aufgebracht auf einem Träger, und Alkylcarbonyloxy- bzw. Alkoxycarbonyloxybenzoesäure und Anhydrid Dibenzylether zugibt und nach Beendigung der Reaktion den Katalysator durch z.B. Filtration oder Zentrifugieren abtrennt.

Eine weitere bevorzugte Durchführungsform ist das Gleichstromverfahren, bei der Dibenzylether und Alkylcarbonyloxy- bzw Alkoxycarbonyloxybenzoesäure und Anhydrid im Gleichstrom beispielsweise von oben her auf eine in einem Rohr angeordnete Katalysatorschüttung aufgebracht und unten am Fuß des Rohres Hydroxybenzoesäurebenzylester abgezogen werden.

In einer weiteren bevorzugten Ausführungsform des erfindungsgemäßen Verfahrens wird dieses in der Rieselphase durchgeführt und der heterogene Katalysator, vorzugsweise eine geträgerte Säure, liegt als Festbettkatalysator vor. Bevorzugt befindet sich die Katalysatorschüttung in einem senkrecht stehenden Rohrreaktor, welcher vorzugsweise Zwischenböden zur besseren Verteilung des Flüssigkeitsstroms und zur besseren Benetzung der Katalysatorschüttung enthält.

Die Aufarbeitung kann sowohl im Falle des suspendierten Katalysators als auch bei Festbettverfahrensvarianten so durchgeführt werden, dass ein mit Wasser nicht mischbares Lösungsmittel, vorzugsweise Toluol, zu den Reaktionsprodukten gegeben wird. Nach der Abtrennung der organischen Phase, welche den rohen Hydroxybenzoesäurebenzylester enthält, kann diese beispielsweise durch Destillation weiter gereinigt werden.

Das erfindungsgemäße Verfahren kann diskontinuierlich, kontinuierlich oder teilkontinuierlich durchgeführt werden.

Die Temperatur, bei das erfindungsgemäße Verfahren durchgeführt wird, beträgt vorzugsweise 15 bis 200, besonders bevorzugt 25 bis 190, ganz besonders bevorzugt 30 bis 180°C.

Bei der Durchführung der Reaktion oberhalb 115°C muß entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Gegebenenfalls kann das erfindungsgemäße Verfahren unter einem üblichen Schutzgas wie beispielsweise Stickstoff, Helium oder Argon, durchgeführt werden.

Das erfindungsgemäße Verfahren kann durch die folgende Reaktionsgleichung erläutert werden:

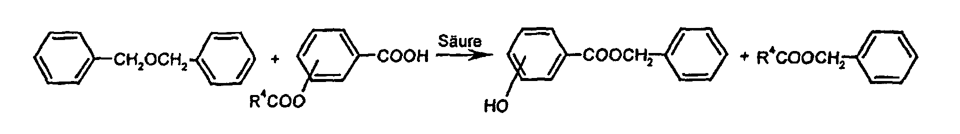

Nach dem erfindungsgemäßen Verfahren erhält man Hydroxybenzoesäurebenzylester in guten Ausbeuten bei hohem Umsatz und guter Selektivität. Das erfindungsgemäße Verfahren ist ohne hohen apparativen Aufwand einfach durchführbar.

Die Prozentangaben der nachfolgenden Beispiele beziehen sich auf das Gewicht.

### Beispiele

### Beispiel 1

49,6 g (0,25 mol) Dibenzylether, 45,0 g (0,25 mol) 2-Acetoxybenzoesäure (2-Acetylsalicylsäure) (Firma Acros) und 0,25 g konzentrierte Schwefelsäure (Riedel-de Haen) wurden in einem Kolben mit Strombrecher und Flügelrührer unter kräftigem Rühren (250 U/min) und unter Stickstoff bei 120°C erhitzt. Nach 3 Stunden Reaktionszeit wurde rasch abgekühlt, die organische Phase nach Zugabe von Toluol und Wasser abgetrennt und gaschromatographisch analysiert. Das Reaktionsgemisch enthält Dibenzylether/Benzylsalicylat/Benzylacetat/2-Acetylbenzylsalicylat im Verhältnis 1,8:40,2:37,3:2,4 , entsprechend einem Umsatz an Dibenzylether von 98 %.

### Beispiel 2

Beispiel 1 wurde wiederholt, aber mit 0,5 g konzentrierter Schwefelsäure (Firma Riedel-de Haen). Nach 0,5 Stunden enthält das Reaktionsgemisch Dibenzylether/Benzylsalicylat/Benzylacetat/2-Acetylbenzylsalicylat im Verhältnis 0,2:37,5:33,6:1,7, entsprechend einem Umsatz von 99 %.

### Beispiel 3

Beispiel 1 wurde wiederholt, aber mit 90,0 g (0,5 mol) 2-Acetoxybenzoesäure und 0,5 g konzentrierter Schwefelsäure (Firma Riedel-de-Haen) bei 90°C. Nach 5 Stunden Reaktionszeit enthält das Reaktionsgemisch Dibenzylether/Benzylsalicylat/Benzylacetat/2-Acetylbenzylsalicylat im Verhältnis 6,2:17,6:25,5:25,0, entsprechend einem Umsatz von 93 %.

### Beispiel 4

Beispiel 1 wurde wiederholt, aber mit 0,1 g Trifluormethansulfonsäure (Firma Acros) bei 90°C. Nach 0,5 Stunden Reaktionszeit enthält das Reaktionsgemisch Dibenzylether/Benzylsalicylat/Benzylacetat/2-Acetylbenzylsalicylat im Verhältnis 6,9:26,5:36,1:1,1, entsprechend einem Umsatz von 93 %.

### Beispiel 5

Beispiel 1 wurde wiederholt, aber mit 0,25 g Trifluormethansulfonsäure-Zinksalz (Firma Fluka). Nach 1 Stunde Reaktionszeit enthält das Reaktionsgemisch Dibenzylether/Benzylsalicylat/Benzylacetat/2-Acetylbenzylsalicylat im Verhältnis 10,3:28,6:33,4:2,2, entsprechend einem Umsatz von 87 %.

### Beispiel 5

Beispiel 1 wurde wiederholt, aber mit 0,5 g Bortrifluoriddietherat (Firma Fluka). Nach 7 Stunden Reaktionszeit enthält das Reaktionsgemisch Dibenzylether/Benzylsalicylat/Benzylacetat/2-Acetylbenzylsalicylat im Verhältnis 15,7:30,6:32,2:11,9, entsprechend einem Umsatz von 81 %.

### Beispiel 6

Beispiel 1 wurde wiederholt, aber mit 0,25 g Molybdatophosphorsäure (Firma Aldrich). Nach 1 Stunde Reaktionszeit enthält das Reaktionsgemisch Dibenzylether/Benzylsalicylat/Benzylacetat/2-Acetylbenzylsalicylat im Verhältnis 68,7:3,5:5,0:3,5, entsprechend einem Umsatz von 9 %.

### Beispiel 7

Beispiel 1 wurde wiederholt, aber mit 0,25 g eines sulfonylierten Styrol-Divinylbenzol-Copolymerisat Lewatit® SC 102 (Firma Bayer). Nach 1 Stunde Reaktionszeit enthält das Reaktionsgemisch Dibenzylether/Benzylsalicylat/Benzylacetat/2-Acetylbenzylsalicylat im Verhältnis 7,1:16,9:21,9:0,3, entsprechend einem Umsatz von 92 %.

## Patentansprüche

1. Verfahren zur Herstellung von Hydroxybenzoesäurebenzylestern der Formel 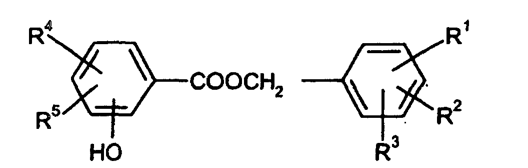 worin
R¹, R² und R³ gleich oder verschieden sind und für Wasserstoff, C₁-C₆-Alkyl C₁-C₆-Alkoxy-, C₁-C₆-Halogenalkyl, C₁-C₆Halogenalkoxy-, CN, CO(C₁-C₆-Alkyl), NO₂ oder Halogen stehen,
R⁴ und R⁵ gleich oder verschieden sind und für
C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen stehen,
aus Dibenzylethern, **dadurch gekennzeichnet, dass** Dibenzylether der Formel 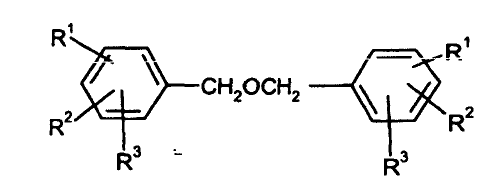 worin
R¹, R² und R³ die oben genannte Bedeutung haben,
oder Gemische aus Dibenzylethern und Benzylalkoholen der Formel 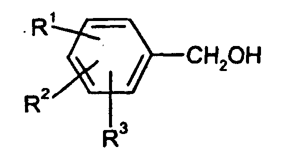 worin
R¹, R² und R³ die oben genannte Bedeutung haben,
mit Verbindungen der Formel 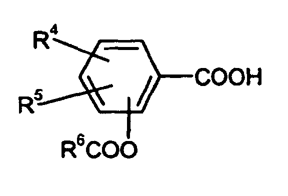 worin
R⁴ und R⁵ die oben genannte Bedeutung haben und
R⁶ für Wasserstoff oder eine geradkettige, verzweigte, gesättigte oder ungesättigte, gegebenenfalls mit Halogen substituierte Alkyl-, Aralkyl-, Aryl-, Alkoxy-, Aralkoxy- und Aryloxygruppe mit 1 bis 50 C-Atomen steht,
in Gegenwart einer oder mehrerer Säure als Katalysator, umgesetzt werden.

2. Verfahren nach Anspruch 1, **dadurch gekennzeichnet, dass** die Säuren sowohl anorganische Säuren, organische Säuren oder Lewissäuren mit einem p_{H}-Wert von 1 bis 6 sind.

3. Verfahren nach den Ansprüchen 1 und 2, **dadurch gekennzeichnet, dass** die Säuren saure Ionenaustauscher sind.

4. Verfahren nach den Ansprüchen 1 bis 3, **dadurch gekennzeichnet, dass** als saurer Ionenaustauscher sulfonylierte Polymere eingesetzt werden.

5. Verfahren nach den Ansprüchen 1 bis 4, **dadurch gekennzeichnet, dass** als saurer Ionenaustauscher fluorierte oder perfluorierte sulfonylierte Polymere, fluorierte oder perfluorierte sulfonylierte Styrol-Divinylbenzol-Copolymerisaten und/oder fluorierte oder perfluorierte sulfonylierte Phenolformaldehyd-Harzen eingesetzt werden.

6. Verfahren nach den Ansprüchen 1 bis 5, **dadurch gekennzeichnet, dass** als Säuren Heteropolysäuren der Formel
Hₐ X_{b} M_{c} O_{d}
worin
H für Wasserstoff und/oder Metallkationen steht,
X für Phosphor, Silizium, Bor oder Germanium steht,
M für Wolfram, Molybden, Vanadium oder Chrom steht und
a für die Zahlen 3, 4, 5 oder 6 steht, mit der Massgabe, dass die Elektroneutralität der Heteropolysäuren oder deren Salze gegeben ist,
b für die Zahlen 1 oder 2 steht,
c für die Zahlen 12 oder 18 steht und
d für die Zahlen 40 oder 62 steht,
eingesetzt werden.

7. Verfahren nach den Ansprüchen 1 bis 6, **dadurch gekennzeichnet, dass** als Heteropolysäure Wolframatophosphorsäure, Wolframatokieselsäure, Molybdatophorsäure und Molybdatokieselsäure eingesetzt werden.

8. Verfahren nach den Ansprüchen 1 bis 7, **dadurch gekennzeichnet, dass** der Dibenzylether unsubstituiert ist.

9. Verfahren nach den Ansprüchen 1 bis 7, **dadurch gekennzeichnet, dass** der Dibenzylether ein substituierter Dibenzylether der Formel 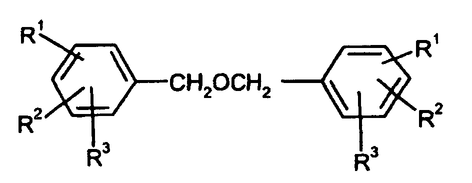 worin
R¹ bis R³ gleich oder verschieden sind und C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen bedeuten.

10. Verfahren nach den Ansprüchen 1 bis 9, **dadurch gekennzeichnet, dass** 0,5 bis 50 mol an Acyloxybenzoesäure, bezogen auf Dibenzylether, eingesetzt werden.

11. Verfahren nach den Ansprüchen 1 bis 10, **dadurch gekennzeichnet, dass** die Umsetzung in Gegenwart von wasserentziehenden Mitteln stattfindet.

12. Verfahren nach den Ansprüchen 1 bis 11, **dadurch gekennzeichnet, dass** die Umsetzung unter Entfernung von Wasser durch Destillation oder Durchleiten von Stickstoff stattfindet.

13. Verfahren nach den Ansprüchen 1 bis 12, **dadurch gekennzeichnet, dass** die Umsetzung in Gegenwart des entsprechenden Anhydrids der eingesetzten Alkylcarbonyloxy- bzw. Alkoxycarbonyloxybenzoesäure durchgeführt wird.

14. Verfahren nach den Ansprüchen 1 bis 13, **dadurch gekennzeichnet, dass** 0,1 bis 10 mol Anhydrid bezogen auf Dibenzylether eingesetzt werden.

15. Verfahren nach den Ansprüchen 1 bis 14, **dadurch gekennzeichnet, dass** die Umsetzung im Temperaturbereich von 15 bis 200°C durchgeführt wird.

16. Verfahren nach den Ansprüchen 1 bis 15, **dadurch gekennzeichnet dass** ein oder mehrere Säuren aus der Gruppe der anorganische, organische oder Lewissäuren ausgewählt werden und in einer Menge von 0,05 bis 10 Gew.-%, bezogen auf die Menge des Dibenzylethers, bzw. mit Belastungen von 1,0 bis 3000 g pro Liter heterogenisierte Säure pro Stunde bei der Anordnung als Festbettkatalysator eingesetzt werden.

17. Verfahren nach den Ansprüchen 1 bis 16, **dadurch gekennzeichnet dass** ein oder mehrere saure Ionenaustauscher ausgewählt aus der Gruppe der, gegebenenfalls fluorierte, sulfonylierte Polystyrole, gegebenenfalls fluorierte oder perfluorierte, sulfonylierte Styrol-Divinylbenzol-Copolymerisaten und gegebenenfalls fluorierte oder perfluorierte sulfonylierte Phenolformaldehyd-Harzen, in einer Menge von 0,5 bis 100 Gew.-%, bezogen auf die Menge des Dibenzylethers, b ei suspendiertem K atalysator bzw. m it B elastungen von 1,0 bis 3000 g Dibenzylether pro Liter Ionenaustauscher pro Stunde bei der Anordnung als Festbettkatalysator eingesetzt werden.

18. Verfahren nach den Ansprüchen 1 bis 17, **dadurch gekennzeichnet dass** ein oder mehrere Heteropolysäuren in einer Menge von 0,05 bis 100 Gew.-%, bezogen auf die Menge des Dibenzylethers, bei suspendiertem Katalysator bzw. mit Belastungen von 1,0 bis 3 000 g D ibenzylether p ro L iter h eterogenisierte Heteropolysäure pro Stunde bei der Anordnung als Festbettkatalysator eingesetzt wird.

## Claims

1. Process for the preparation of hydroxybenzoic benzyl esters of the formula 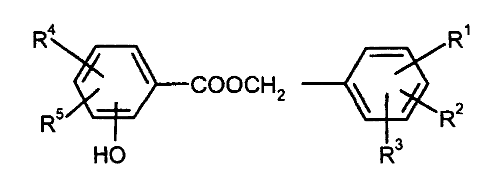 in which
R¹, R² and R³ are identical or different and are hydrogen, C₁-C₆-alkyl, C₁-C₆-alkoxy-, C₁-C₆-halogenoalkyl, C₁-C₆-halogenoalkoxy-, CN, CO(C₁-C₆-alkyl), NO₂ or halogen,
R4 and R5 are identical or different and are
C₁-C₆-alkyl, C₁-C₆-alkoxy-, C₁-C₆-halogenoalkyl, C₁-C₆-halogenoalkoxy-, CN, CO(C₁-C₆-alkyl), NO₂ or halogen,
from dibenzyl ethers, **characterized in that** dibenzyl ethers of the formula 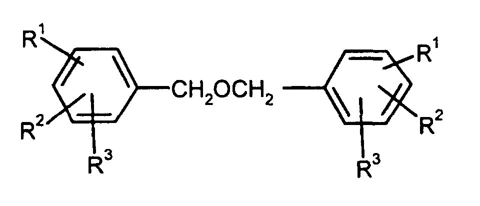 in which
R¹, R² and R³ are as defined above,
or mixtures of dibenzyl ethers and benzyl alcohols of the formula 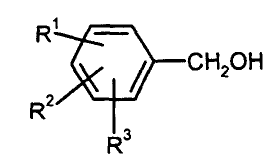 in which
R¹, R² and R³ are as defined above,
are reacted with compounds of the formula 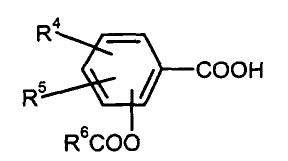 in which
R⁴ and R⁵ are as defined above and
R⁶ is hydrogen or a straight-chain, branched, saturated or unsaturated, optionally halogen-substituted alkyl, aralkyl, aryl, alkoxy, aralkoxy and aryloxy group having 1 to 50 carbon atoms,
in the presence of one or more acids as catalyst.

2. Process according to Claim 1, **characterized in that** the acids are either inorganic acids, organic acids or Lewis acids with a p_{H} value of from 1 to 6.

3. Process according to Claims 1 and 2, **characterized in that** the acids are acidic ion exchangers.

4. Process according to Claims 1 to 3, **characterized in that** the acidic ion exchangers used are sulphonylated polymers.

5. Process according to Claims 1 to 4, **characterized in that** the acidic ion exchangers used are fluorinated or perfluorinated sulphonylated polymers, fluorinated or perfluorinated sulphonylated styrene-divinylbenzene copolymers and/or fluorinated or perfluorinated sulphonylated phenol-formaldehyde resins.

6. Process according to Claims 1 to 5, **characterized in that** the acids used are heteropolyacids of the formula
Hₐ X_{b} M_{c} O_{d}
in which
H is hydrogen and/or metal cations,
X is phosphorus, silicon, boron or germanium,
M is tungsten, molybdenum, vanadium or chromium, and
a is 3, 4, 5 or 6, with the proviso that the heteropolyacids or salts thereof have electroneutrality,
b is 1 or 2,
c is 12 or 18, and
d is 40 or 62.

7. Process according to Claims 1 to 6, **characterized in that** the heteropolyacid used is phosphotungstic acid, silicotungstic acid, phosphomolybdic acid or silicomolybdic acid.

8. Process according to Claims 1 to 7, **characterized in that** the dibenzyl ether is unsubstituted.

9. Process according to Claims 1 to 7, **characterized in that** the dibenzyl ether is a substituted dibenzyl ether of the formula 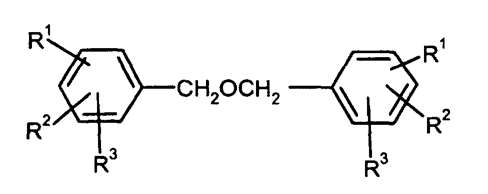 in which
R¹ to R³ are identical or different and are C₁-C₆-alkyl, C₁-C₆-alkoxy-, C₁-C₆-halogenoalkyl, C₁-C₆-halogenoalkoxy-, CN, CO(C₁-C₆-alkyl), NO₂ or halogen.

10. Process according to Claims 1 to 9, **characterized in that** 0.5 to 50 mol of an acyloxybenzoic acid, based on dibenzyl ether, are used.

11. Process according to Claims 1 to 10, **characterized in that** the reaction is carried out in the presence of water-withdrawing agents.

12. Process according to Claims 1 to 11, **characterized in that** the rection is carried out with the removal of water by distillation or by passing nitrogen through.

13. Process according to Claims 1 to 12, **characterized in that** the reaction is carried out in the presence of the corresponding anhydride of the alkylcarbonyloxybenzoic or alkoxycarbonyloxybenzoic acid used.

14. Process according to Claims 1 to 13, **characterized in that** 0.1 to 10 mol of anhydride, based on dibenzyl ether, are used.

15. Process according to Claims 1 to 14, **characterized in that** the reaction is carried out in the temperature range from 15 to 200°C.

16. Process according to Claims 1 to 15, **characterized in that** one or more acids are chosen from the group of inorganic, organic or Lewis acids and are used in an amount of from 0.05 to 10% by weight, based on the amount of the dibenzyl ether, or with space velocities of from 1.0 to 3000 g per litre of heterogenized acid per hour if the catalyst is arranged as a fixed bed.

17. Process according to Claims 1 to 16, **characterized in that** one or more acidic ion exchangers chosen from the group of optionally fluorinated, sulphonylated polystyrenes, optionally fluorinated or perfluorinated, sulphonylated styrene-divinylbenzene copolymers and optionally fluorinated or perfluorinated sulphonylated phenol-formaldehyde resins, are used in an amount of from 0.5 to 100% by weight, based on the amount of dibenzyl ether, in the case of a suspended catalyst, or with space velocities of from 1.0 to 3000 g of dibenzyl ether per litre of ion exchanger per hour if the catalyst is arranged as a fixed bed.

18. Process according to Claims 1 to 17, **characterized in that** one or more heteropolyacids are used in an amount of from 0.05 to 100% by weight, based on the amount of dibenzyl ether, in the case of a suspended catalyst, or with space velocities of from 1.0 to 3000 g of dibenzyl ether per litre of heterogenized heteropolyacid per hour if the catalyst is arranged as a fixed bed.

## Revendications

1. Procédé de préparation d'esters benzyliques d'acides hydroxybenzoïques de formule 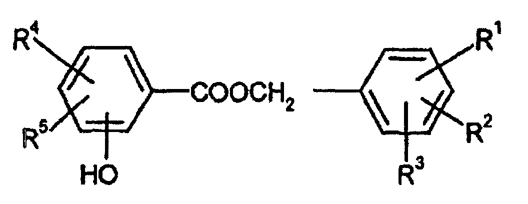 dans laquelle
R¹, R² et R³ sont identiques ou différents et représentent un atome d'hydrogène, un groupe alkyle en C₁ à C₆, un groupe alcoxy en C₁ à C₆, un groupe halogénoalkyle en C₁ à C₆, un groupe halogénoalcoxy en C₁ à C₆, CN, CO(alkyle en C₁ à C₆), NO₂ ou un atome d'halogène,
R⁴ à R⁵ sont identiques ou différents et représentent
un groupe alkyle en C₁ à C₆, un groupe alcoxy en C₁ à C₆, un groupe halogénoalkyle en C₁ à C₆, un groupe halogénoalcoxy en C₁ à C₆, CN, CO(alkyle en C₁ à C₆), NO₂ ou un atome d'halogène,
à partir d'éthers dibenzyliques, **caractérisé en ce que** des éthers dibenzyliques de formule 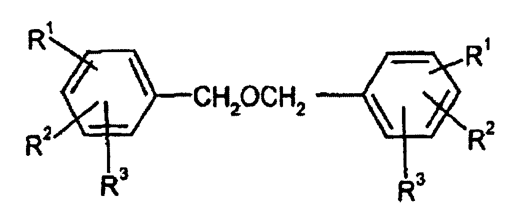 dans laquelle
R¹, R² et R³ ont la signification précédemment citée,
ou des mélanges d'éthers dibenzyliques et d'alcools benzyliques de formule 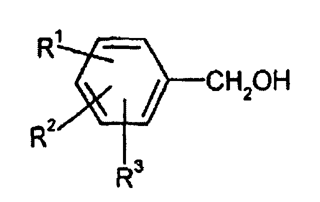 dans laquelle
R¹, R² et R³ ont la signification précédemment citée,
sont mis en réaction avec des composés de formule 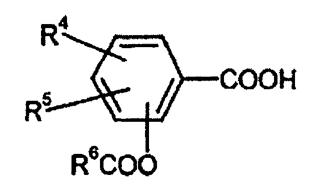 dans laquelle
R⁴ et R⁵ ont la signification précédente et
R⁶ représente un atome d'hydrogène ou un groupe alkyle, aralkyle, aryle, alcoxy, aralcoxy ou aryloxy à chaîne droite, ramifié, saturé ou non saturé, le cas échéant substitué par un atome d'halogène et comprenant 1 à 50 atomes de carbone,
en présence d'un ou de plusieurs acides, comme catalyseur.

2. Procédé selon la revendication 1, **caractérisé en ce que** les acides sont aussi bien des acides inorganiques, organiques ou que des acides de Lewis présentant un pH de 1 à 6.

3. Procédé selon les revendications 1 et 2, **caractérisé en ce que** les acides sont des échangeurs d'ions acides.

4. Procédé selon les revendications 1 à 3, **caractérisé en ce que** l'on utilise comme échangeurs d'ions acides des polymères sulfonylés.

5. Procédé selon les revendications 1 à 4, **caractérisé en ce que** l'on utilise comme échangeurs d'ions acides des polymères sulfonylés perfluorés ou fluorés, des copolymères de divinylbenzène-styrène sulfonylés perfluorés ou fluorés et/ou des résines de phénolformaldéhyde sulfonylées perfluorées ou fluorées.

6. Procédé selon les revendications 1 à 5, **caractérisé en ce que**, l'on utilise comme acides des hétéropolyacides de formule
HₐX_{b}M_{c}O_{d}
dans laquelle
H représente un atome d'hydrogène et/ou des cations de métal,
X représente le phosphore, le silicium, le bore ou le germanium,
M représente le tungstène, le molybdène, le vanadium ou le chrome et
a représente les nombres 3, 4, 5 ou 6, à la condition que l'électroneutralité soit donnée aux hétéropolyacides ou à leurs sels,
b représente les nombres 1 ou 2,
c représente les nombres 12 ou 18 et
d représente les nombres 40 ou 62.

7. Procédé selon les revendications 1 à 6, **caractérisé en ce que** l'on utilise comme hétéropolyacides des acides tungstatophosphoriques, des acides tungstatosiliciques, des acides molybdatophosphoriques et des acides molybdatosiliciques.

8. Procédé selon les revendications 1 à 7, **caractérisée en ce que** l'éther dibenzylique n'est pas substitué.

9. Procédé selon les revendications 1 à 7, **caractérisée en ce que** l'éther dibenzylique est un éther dibenzylique substitué de formule 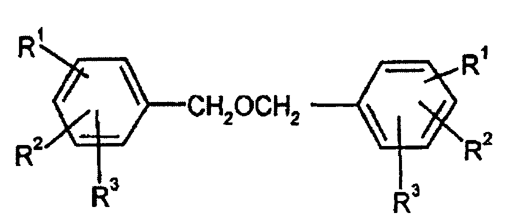 dans laquelle
R¹ à R³ sont identiques ou différents et représentent un groupe alkyle en C₁ à C₆, un groupe alcoxy en C₁ à C₆, un groupe halogénoalkyle en C₁ à C₆, un groupe halogénoalcoxy en C₁ à C₆, CN, CO(alkyle en C₁ à C₆), NO₂ ou un atome d'halogène.

10. Procédé selon les revendications 1 à 9, **caractérisé en ce que** l'on utilise 0,5 à 50 moles d'un acide acyloxybenzoïque, rapportées à l'éther dibenzylique.

11. Procédé selon les revendications 1 à 10, **caractérisé en ce que** la conversion est réalisée en présence d'un agent de suppression de l'eau.

12. Procédé selon les revendications 1 à 11, **caractérisé en ce que** la conversion a lieu en supprimant l'eau par distillation ou par barbotage de l'azote.

13. Procédé selon les revendications 1 à 12, **caractérisé en ce que** la conversion est réalisée en présence d'anhydride correspondant de l'acide alkylcarbonyloxybenzoïque ou alcoxycarbonyloxybenzoïque utilisé.

14. Procédé selon les revendications 1 à 13, **caractérisé en ce que** l'on utilise 0,1 à 10 moles d'anhydride rapportées à l'éther dibenzylique.

15. Procédé selon les revendications 1 à 14, **caractérisé en ce que** la conversion est réalisée dans une plage de températures de 15 à 200°C.

16. Procédé selon les revendications 1 à 15, **caractérisé en ce qu'**un ou plusieurs acides sont choisis parmi le groupe des acides organiques, inorganiques ou des acides de Lewis et sont utilisés dans une quantité de 0,05 à 10 % en poids, rapportée à la quantité d'éther dibenzylique, ou avec des charges de 1,0 à 3 000 g par litre d'acide hétérogénéisé par heure lors de l'agencement sous la forme d'un catalyseur à lit fixe.

17. Procédé selon les revendications 1 à 16, **caractérisé en ce qu'**un ou plusieurs échangeurs d'ions acides sont choisis dans le groupe se composant de polystyrènes sulfonylés, le cas échéant fluorés, de copolymères de divinylbenzène-styrène sulfonylés, le cas échéant fluorés ou perfluorés et des résines de phénolformaldéhyde sulfonylées, le cas échéant fluorées ou perfluorées dans une quantité de 0,5 à 100 % en poids, rapportée à la quantité d'éther dibenzylique, pour un catalyseur en suspension ou avec des charges de 1,0 à 3 000 g d'éther dibenzylique par litre d'échangeur d'ions par heure lors de l'agencement sous la forme d'un catalyseur à lit fixe.

18. Procédé selon les revendications 1 à 17, **caractérisé en ce qu'**un ou plusieurs hétéropolyacides sont utilisés dans une quantité de 0,05 à 100 % en poids, rapportée à la quantité d'éther dibenzylique, pour le catalyseur en suspension ou avec des charges de 1,0 à 3 000 g d'éther dibenzylique par litre d'hétéropolyacide hétérogénéisé par heure lors de l'agencement sous la forme d'un catalyseur à lit fixe.
